# EUROPÄISCHE PATENTSCHRIFT

(11) **EP 2 627 313 B1**
(45) Veröffentlichungstag und Bekanntmachung des Hinweises auf die Patenterteilung: **12.09.2018**
(21) Anmeldenummer: 11770057.5
(22) Anmeldetag: 11.10.2011
(51) Int. Cl.: A61K 31/785, A61P 13/12, A61K 9/46, A61K 9/00, A61K 9/16, A61K 33/06, A61K 33/10

(54) **PHOSPHATBINDERFORMULIERUNG ZUR EINFACHEN EINNAHME**
PHOSPHATE BINDER FORMULATION FOR SIMPLE INGESTION
FORMULATION CONTENANT DES AGENTS D'AGGLUTINATION DU PHOSPHATE PERMETTANT UNE MEILLEURE ADMINISTRATION

(30) Priorität: 13.10.2010 EP 10013578
(43) Veröffentlichungstag der Anmeldung: 21.08.2013
(73) Patentinhaber: Fresenius Medical Care Deutschland GmbH, 61352 Bad Homburg (DE)
(72) Erfinder: STROTHMANN, Kai, 52428 Jülich (DE); SCHULZE, Friedrich, 55286 Wörrstadt (DE); BARTHOLOMÄUS, Johannes, 52080 Aachen (DE); FRIES-SCHAFFNER, Eva, 61118 Bad Vilbel (DE); OPPERMANN, Astrid, 60488 Frankfurt (DE)
(74) Vertreter: Weiß, Stefan
(86) Internationale Anmeldenummer: PCT/EP2011/005069
(87) Internationale Veröffentlichungsnummer: WO 2012/048846

(56) Entgegenhaltungen:
- EP-A1- 0 424 706
- EP-A1- 0 525 388
- WO-A2-2007/088343
- US-A1- 2006 121 127
- US-A1- 2007 059 277

## Beschreibung

### Ausgangssituation und Stand der Technik:

Die Erfindung betrifft pharmazeutische Zusammensetzungen, enthaltend Phosphatbinder, die oral, ohne den Zusatz von Wasser, eingenommen werden können. Die pharmazeutische Zusammensetzung liegt dabei in Form eines Granulats vor und enthält ein Brausemittel.

Niereninsuffiziente Patienten und insbesondere Dialysepatienten leiden häufig an einer behandlungsbedürftigen Hyperphosphatämie. Eine Hyperphosphatämie ist eine pathophysiologische Erhöhung des Phosphatspiegels im Blut. Eine Hyperphosphatämie entsteht bei massiver Phosphatzufuhr, massiver Freisetzung von Phosphat durch Gewebszerstörung, verminderter Phosphatausscheidung bei fortgeschrittenem Nierenversagen und/oder Zuständen mit einer gesteigerten Phosphatrückresorption durch die Nieren. Der Anstieg des Serum-Phosphats kann zur Ablagerung von Calciumphosphat in Blutgefäßen und anderen Geweben führen. Die chronische Hyperphosphatämie kann zu Verkalkungen von Geweben und Blutgefäßen führen, die in Durchblutungsstörungen, Herzinfarkt und/oder Schlaganfall resultieren können. Niereninsuffiziente Patienten müssen deshalb meist Phosphatbinder einnehmen, um Ihren Serumphosphatspiegel zu kontrollieren. Die Phosphatbinder sollen mit den Mahlzeiten eingenommen werden, um das in der Nahrung enthaltene Phosphat im Darm in Form schwerlöslicher Salze oder nicht resorbierbarer Komplexe zu binden und so mit den Faeces zur Ausscheidung zu bringen. Dazu müssen die Patienten eine große Menge des Phosphatbinders in der Regel in Form einer größeren Zahl von Tabletten einnehmen, zum Beispiel drei mal täglich zu jeder Mahlzeit 1-5 Tabletten. Um die entsprechend große Menge leichter oder mit weniger Tabletten einnehmen zu können, müssen entweder im Falle kleinerer Tabletten eine noch größere Zahl oder im Falle von weniger Tabletten dafür wesentlich größere, und schwerer einnehmbare Tabletten verabreicht werden. Diese große Anzahl an Tabletten ist jedoch nur ein Teil der oralen Gesamtmedikation eines niereninsuffizienten Patienten. So erhielten in einer aktuellen Untersuchung an 233 Dialysepatienten diese im Durchschnitt 11 verschiedene Medikamente, was im Median zu 19 einzunehmenden Tabletten pro Tag führte (Chiu YW. 2009). Hiervon waren ca. 50% Phosphatbinder.

Hinzu kommt, dass niereninsuffiziente Patienten und insbesondere Dialysepatienten nur wenig Wasser pro Tag aufnehmen dürfen, so dass die Tabletten nicht gut geschluckt werden können. Niereninsuffiziente Patienten können überschüssige Flüssigkeit im Körper nicht oder nur eingeschränkt über den Urin ausscheiden. Daher leiden die meisten niereninsuffizienten Patienten an einem permanenten Flüssigkeitsüberschuss im Körper, der Wohlbefinden und Mortalität negativ beeinflusst. Ein Flüssigkeitsüberschuss kann Bluthochdruck bewirken, der das kardiovaskuläre System und insbesondere die Funktion der linken Herzkammer schädigen kann und somit indirekt zu einer erhöhten Mortalität von niereninsuffizienten Patienten durch kardiovaskuläre Krankheiten beiträgt. Ferner leidet ein großer Anteil der niereninsuffizienten Patienten an Anämie und erhält eine blutbildungsfördernde Medikation in Form von Epogen oder Analoga und in Form von Eisenpräparaten. Ein Flüssigkeitsüberschuss verringert die Konzentration der blutbildungsfördernden Medikamente und erschwert damit eine geeignete Dosierung dieser Medikamente erheblich. Daher ist die Regulierung der Flüssigkeitseinnahme bei niereninsuffizienten Patienten von größter Wichtigkeit. In den meisten Fällen ist es das Ziel, die Flüssigkeitszufuhr bei niereninsuffizienten Patienten zu minimieren.

Erschwerend kommt hinzu, dass der Speichelfluss bei Dialysepatienten weniger als halb so stark im Vergleich zu gesunden Kontrollgruppen ist (Bayraktar G. 2004). Daher ist die Compliance der Einnahme negativ beeinflusst. Im Gegensatz zur Anzahl der verschriebenen unterschiedlichen Medikamente steht vor allem die Zahl der verschriebenen Phosphatbinder-Tabletten in Zusammenhang mit der Compliance der Patienten (Arenas MD. 2010) Befragt man Dialysepatienten, welche Medikamente sie am liebsten weglassen würden, wenn sie die Wahl hätten, so werden an erster Stelle die Phosphatbinder genannt (Arenas MD. 2010). Maßgeblich würden eine verbesserte, einnahmefreundlichere Arzneiform und ein besserer Geschmack der Phosphatbinder den Wünschen der Patienten entgegenkommen (Lindberg M. 2008).

US 7,465,465 offenbart zur Abhilfe des Problems Kautabletten mit Lanthan als Phosphatbinder, die vor dem Schlucken zerkaut werden müssen. Dies kann dem Dialysepatienten, der einen verminderten Speichelfluss aufweist, schwer fallen. Das Zerkleinern der Tablette und anschließendes Verteilen auf Nahrung oder einen Löffel ist mit der Gefahr der Mindereinnahme an Wirkstoff aufgrund von Verlusten bei der Herrichtung beziehungsweise der unvollständigen Einnahme der Nahrung verbunden.

EP 1 924 246 offenbart Pulverzubereitungen mit einem Suspensionsstabilisator zur Einnahme der Pulver nach Suspendieren in Wasser. Dies ist mit dem Nachteil der höheren Wassermenge der Suspension entgegen der Wasserrestriktionen für den Dialysepatienten verbunden. Es ist üblich, ein solches Pulver in mindestens 40 ml - 60 ml Wasser vor der Einnahme zu suspendieren. So summiert sich allein für die Phosphatbindergabe die aufgenommene Wassermenge auf ca. 120 - 300 ml pro Tag. Um die Gesamtdosis des Phosphatbinders sicher zuzuführen, ist es weiterhin erforderlich, am Rand anhaftende Pulverpartikel, die nach der Einnahme an der Glaswand verblieben sind, mit weiterem Wasser abzuspülen und dieses ebenfalls einzunehmen.

Zur Reduktion der Wassereinnahme mit dem Phosphatbinder offenbart WO 2008/011126 konzentrierte Lösungen von Calciumacetat mit hohen Mengen Polyolen, Süßmitteln und geschmacksmaskierenden Substanzen. In solchen Formulierungen bleibt einerseits immer noch ein Anteil des schlechten Eigengeschmacks des Calciumacetats spürbar und andererseits lehnt eine Reihe von Patienten den intensiven, immer gleichbleibenden Geschmack der Süßmittel und Aromen, die nicht mit jeder Mahlzeit korrespondieren, ab. Für Kombinationsprodukte mit leicht wasserlöslichem Calciumacetat und schwerlöslichem Magnesiumcarbonat ergibt sich fernerhin der Nachteil einer Dosierungsungenauigkeit, da Magnesium in suspendierter Form schwierig mittels Tropfer usw. zu dosieren ist.

Alle beschriebenen Lösungsansätze zur einfacheren Einnahme weisen ferner den Nachteil auf, dass sich in jedem Fall das Produkt in weiten Bereichen des Mundraums unwillkürlich verteilt und so einen unerwünschten Geschmack erzeugt. Intensiver wird dieser Geschmack, wenn Teile des Produkts in Teilen des Mundraums auch noch eine längere Verweilzeit aufweisen.

Es ist daher die Aufgabe der Erfindung eine Applikationsform für Phosphatbinder zur Verfügung zu stellen, die es niereninsuffizienten Patienten erlaubt, die notwendigen großen Mengen Phosphatbinder einfach und ohne zusätzliche Wassermengen einzunehmen. In einem zweiten Aspekt sollen die Arzneiformen einen eventuellen schlechten Geschmack des Phosphatbinders maskieren und ein angenehmes Mundgefühl vermitteln. In einem weiteren Aspekt ist es Aufgabe der Erfindung eine Zusammensetzung zur Verfügung zu stellen, die sich möglichst wenig unwillkürlich im Mundraum verteilt und eine exakte Dosierung erlaubt.

Die Aufgaben werden durch die Zusammensetzung nach Anspruch 1 und deren Anwendung gelöst, sowie durch die Gegenstände der weiteren unabhängigen Ansprüche.

Weitere Aufgaben werden durch bevorzugte Ausführungsformen der Erfindung gelöst, die Gegenstand der abhängigen Ansprüche sind.

Eine pharmazeutische Zusammensetzung in Form eines rieselfähigen Granulats oder einer Kautablette enthaltend mindestens eine phosphatbindende Substanz und mindestens ein Brausemittel, das ein Alkali- oder Erdalkalicarbonat oder ein Alkali- oder Erdalkalihydrogencarbonat und eine feste, organische, essbare Säure oder deren saures Salz enthält, wird in der Behandlung von Hyperphosphatämie bei niereninsuffizienten Patienten angewendet und zeichnet sich dadurch aus, dass das Granulat oral ohne den Zusatz von Wasser oder einer anderen Flüssigkeit appliziert wird und anschließend abgeschluckt werden kann. Auf diese Weise kann eine Einnahme ohne zusätzliche Flüssigkeitszufuhr erfolgen. Ein Nachspülen mit Wasser wird dabei nicht zwingend ausgeschlossen, ist aber nicht erforderlich. Entscheidend ist, dass die Zusammensetzung zunächst wasserfrei im Mundraum aufgenommen wird. Das Brausemittel wirkt dabei geschmacksmaskierend und vermittelt ein angenehmes Mundgefühl. Auf diese Weise kann die pharmazeutische Zusammensetzung mit Speichel angelöst und von der Zunge weitgehend quantitativ abgeschluckt werden.

Idealerweise wird die pharmazeutische Zusammensetzung aus einem Stickpack direkt auf die Zunge appliziert. Im Fall eines schlecht schmeckenden Wirkstoffs kann dieser im Granulat zusätzlich geschmacksmaskierend überzogen sein, wenn eine einfache Aromatisierung und/oder das Brausemittel nicht zur Geschmacksmaskierung ausreichen.

Phosphatbinder sind Stoffe, die durch Wechselwirkung mit Phosphationen einschließlich protonierter Phosphationen diese daran hindern, aus dem Gastrointestinaltrakt in den Blutkreislauf und damit den Körper aufgenommen zu werden, und für die Ausscheidung der Phosphate mit den Faeces sorgen.

Als Phosphatbinder kommen insbesondere in Frage Calcium-, Magnesium-, Aluminium-, Eisen-, Lanthan- und Bismutsalze, deren Löslichkeitsprodukte größer als die der entsprechenden Phosphatsalze dieser Kationen sind. Weiterhin bilden phosphatbindende organische Polymere mit Anionenaustauscherfunktion wie z.B. Sevelamer, AMG 223 (Fa. Amgen) und MCI-196 (Colestilan, Fa. Mitsubishi) geeignete Wirkstoffe für die Erfindung. Als Aluminiumsalze eignen sich alle pharmazeutisch verträglichen Salze, die die obigen Anforderungen erfüllen, besonders bevorzugt Oxide, insbesondere Algedrat, und/oder Hydroxide. Als Lanthansalze eignen sich alle pharmazeutisch verträglichen Salze, die die obigen Anforderungen erfüllen, insbesondere Lanthancarbonat einschließlich seiner Hydrate. Als Magnesiumsalze eignen sich alle pharmazeutisch verträglichen Salze, die die obigen Anforderungen erfüllen, bevorzugt Chloride, Sulfate, Hydroxide, Oxide, Carbonate und insbesondere schweres Magnesiumcarbonat. Bevorzugte Phosphatbinder auf Metallsalzbasis sind Eisenhydroxide, Eisenoxidhydroxide und Eisencitrate, insbesondere solche Eisenpräparate, die durch Kohlenhydrate oder Huminsäure stabilisiert oder daran gebunden sind oder Schichtsalze mit Magnesium bilden, wie zum Beispiel Fermagate sowie Calciumsalze, bevorzugt Calciumcarbonat und/oder Calciumchlorid und besonders bevorzugt Calciumacetat. Calciumacetat wird gegenüber Calciumcarbonat wegen seiner hohen Löslichkeit bevorzugt, hat allerdings den Nachteil eines ausgeprägt unangenehmen Geschmacks. Unter den Metallsalzen sind Calciumsalze besonders gute Phospatbinder und günstig im Preis. Als nachteilig wird allerdings diskutiert, dass Calciumpräparate in manchen Patienten zu einer Hypercalcämie, also einem erhöhten Calciumspiegel im Blut führen können. Chronische Hypercalcämie wird mit dem Risiko einer Calcifizierung der Blutgefäße in Verbindung gebracht und somit wiederum mit dem Risiko von kardiovaskulären Erkrankungen. Der Ersatz von Teilen des Calciums in solchen Phosphatbindern durch Magnesium, welches selber als Phosphatbinder fungiert, verringert das Risiko der Entstehung einer Hypercalcämie. Es konnte gezeigt werden, dass durch den Zusatz von Magnesiumsalzen, insbesondere Magnesiumcarbonat zu phosphatbindendem Calciumacetat die Hypercalcämie und somit die Calzifizierung soweit vermieden werden kann, dass sich keine nachteiligen Wirkung im Vergleich zu mit calciumfreien Phosphatbindern behandelten Patienten nachweisen lassen. (de Francisco ALM et al. Nephrol Dial Transplant 2010). Zur Erzielung dieser Wirkung wird ein bevorzugtes Verhältnis von Calciumacetat zu Magnesiumcarbonat von 1:1 bis 4:1 angenommen, besonders bevorzugt ist ein Verhältnis von 1,2:1 bis:3:1. und ganz besonders bevorzugt eine Verhältnis von 1.5:1 bis 2:1. Magnesiumsalze besitzen darüber hinaus auch eine eigenständige phosphatbindende Wirkung, so dass in Gegenwart von Magnesiumsalzen die benötigte Menge des Phosphatbinders auf Calciumbasis verringert werden kann. Eine Kombination von Calciumacetat und Magnesiumcarbonat stellt somit eine besonders bevorzugte Zusammensetzung im Sinne der Erfindung dar. Ein weiterer besonderer Vorteil dieser Kombination ergibt sich daraus, dass Magnesiumcarbonat auch ein bevorzugter Bestandteil des Brausemittels ist, insofern kann Magnesiumcarbonat innerhalb der Zusammensetzung zwei Funktionen gleichzeitig erfüllen. Auf diese Weise kann das Gewicht und das Volumen der Zusammensetzung pro Dosis, die durch den Patienten einzunehmen ist, weiter verringert werden. Eine Tagesdosis enthält idealerweise 100 mg - 3000 mg Calcium und 0 mg - 1500 mg Magnesium bezogen auf das Gewicht der Metallionen, bevorzugt werden 300 mg - 1800 mg Calcium und 180 mg - 750 mg Magnesium, am bevorzugtesten 450 - 1350 mg Calcium und 180 - 540 mg Magnesium. In Abwesenheit von Magnesium erhöhen sich die bevorzugten Mengen für Calcium um den Faktor 1,5.

Für Ionenaustauscherpolymere, insbesondere Sevelamer, beträgt die ideale Tagesdosis 500 - 12000 mg, bevorzugt 2000 - 10000 mg und besonders bevorzugt 5000 - 8000 mg.

Für Phosphatbinder auf Lanthanbasis beträgt die ideale Tagesdosis bezogen auf Lanthan 250 - 5000 mg, bevorzugt 750 - 4000 mg, 1500 - 3000 mg.

Die Tagesdosis wird üblicherweise auf 3 bis 15 Einzeldosen aufgeteilt, die mit jeder Mahlzeit einzunehmen sind. Zu den drei Hauptmahlzeiten pro Tag ist es üblich 1-4 Einzeldosen einzunehmen.

Wegen der ohnehin großen Menge, in der Phospatbinder eingenommen werden müssen, ist es ein Ziel der Erfindung, das Gewicht und das Volumen der Endzusammensetzung so klein wie möglich zu halten. Die Beimischung weiterer Hilfsstoffe soll daher auf ein Minimum beschränkt werden. Bevorzugt enthält erfindungsgemäße Zusammensetzung 15 bis 80% des phosphatbindenden Wirkstoffs, besonders bevorzugt 20 bis 70% und ganz besonders bevorzugt 25 bis 60%.

Alle genannten Phosphatbinder müssen in großen Mengen eingenommen werden und es ist Aufgabe der Erfindung die Einnahme dieser Mengen ohne zusätzliche Zugabe von Flüssigkeit zu ermöglichen. Tabletten dieser Größe oder Kapseln mit Wirkstoff lassen sich ohne gleichzeitige Einnahme einer Flüssigkeit so gut wie nicht abschlucken. Es verbleiben die Möglichkeiten einer Kautablette oder eines Granulats. Für beide Einnahmeformen ist es von essentieller Bedeutung, dass die Applikationsform wohlschmeckend ist und ein angenehmes Mundgefühl vermittelt, weil beide Applikationsformen anwendungsgemäß im Mundraum eine große Oberfläche haben und das Potential haben, sich im gesamten Mundraum zu verteilen. Unangenehmer Geschmack oder unangenehmes Mundgefühl wird also bei diesen Applikationsformen besonders stark empfunden. Alle genannten Phosphatbinder vermitteln in Form eines Granulats oder einer im Mund zerfallenen Kautablette ein unangenehmes "kalkiges" oder "sandiges" und vor allem trockenes Mundgefühl. Insbesondere das trockene Mundgefühl ist bei niereninsuffizienten Patienten, die oft an mangelnder Speichelbildung leiden, noch verstärkt. Hinzu kommt, dass einige der Wirkstoffe, wie zum Beispiel Calciumacetat, einen sehr unangenehmen Eigengeschmack haben.

Erfindungsgemäß wird der Zusammensetzung daher ein Brausemittel zugesetzt. Das Brausemittel wird im Mundraum durch Speichel angelöst und setzt in einer chemischen Säure-Base-Reaktion Kohlendioxid frei. Das freigesetzte Kohlendioxid regt zum einen weitere Speichelbildung an, insbesondere im Zusammenspiel mit Aromen, und wird darüber hinaus im Mundraum als frisch und angenehm empfunden. Durch die vermehrte Speichelbildung schwindet das Gefühl der Trockenheit und die Zusammensetzung lässt sich vermischt mit dem Speichel leichter abschlucken. Darüber hinaus besitzt das freigesetzte Kohlendioxid in sich eine geschmacksmaskierende Wirkung.

Das Brausemittel enthält mindestens eine feste, organische, essbare Säure und/oder deren saures Salz und mindestens ein Kohlendioxid freisetzendes Salz, insbesondere ein Carbonat, bevorzugt ausgewählt aus der Gruppe der Akalisalze und Erdalkalisalze oder deren Metallhydrogensalze. Besonders geeignet sind Natriumcarbonat, Natriumhydrogencarbonat, Kaliumcarbonat, Kaliumhydrogencarbonat, Magnesiumcarbonat und/oder Calciumcarbonat. Besonders bevorzugt wird Magnesiumcarbonat verwendet, weil es neben der Brausefunktion auch als Wirkstoff gegen Hypercalcämie wirkt und phosphatbindende Eigenschaften hat. Weiterhin bevorzugt wird Calciumcarbonat verwendet, weil das im Brausemittel zugeführte Calcium phosphatbindend wirken kann und somit eine Reduktion des Wirkstoffs ermöglicht. Die Brausereaktion wirkt auch dem Nachteil der geringen Löslichkeit von Calciumcarbonat entgegen. Als Säuren eignen sich besonders Citronensäure, Weinsäure, Äpfelsäure, Adipinsäure, Bernsteinsäure, Fumarsäure, Ascorbinsäure, Maleinsäure und Ascorbinsäure sowie bei mehrbasigen Säuren auch Teilsalze dieser Säuren wie zum Beispiel Mononatriumtartrat. Besonders bevorzugt sind solche Säuren, die den Speichelfluss zusätzlich noch anregen und/oder einen angenehmen Eigengeschmack aufweisen, um zum einen das Abschlucken der leicht schäumenden Zubereitung zu erleichtern und zum anderen den Geschmack der Zusammensetzung zu verbessern. Hierzu eigen sich besonders Weinsäure, Citronensäure, Ascorbinsäure, Natrium- und Kaliumtartrat, Natriumhydrogencitrat oder Natriumascorbat.

Die Herstellung der erfindungsgemäßen Brauseformulierungen kann nach üblichen, im Stand der Technik bekannten Verfahren erfolgen. Beispielsweise werden die Säuren und Carbonate getrennt granuliert, vorzugsweise mittels Feuchtgranulierung, wobei die Wirkstoffe einem der Granulate beigefügt werden können. Nach Mischung der sorgfältig getrockneten Granulate werden bevorzugt lösliche Gleitmittel, wie Natriumbenzoat oder Polyaethylenglykole zugesetzt und komprimiert. Nach einer anderen Methode werden alle Säuren, Carbonate und Wirkstoffe zusammengemischt und im Reaktor erhitzt, bis beispielsweise die Zitronensäure ihr Kristallwasser freigibt und ein Granulat entsteht. Wiederholtes Umrühren ist erforderlich, um eine gleichmäßige Masse zu erhalten. Diese wird dann rasch gesiebt und sorgfältig getrocknet. Ein gutes Trocknen ist unbedingt notwendig, um ein allmähliches Zerfallen des Granulats durch Reaktion der Säuren mit den Carbonaten zu vermeiden. Um eine rasche Trocknung zu erreichen, werden beispielsweise Vakuumtrockenschränke oder sogenannte Eintopfgranulatoren mit Vakuum unterstützter Trocknung verwendet. In einer anderen Variante der Herstellung erfolgt die Anreaktion der Säure mit basischen Bestandteilen mit anschließender Trocknung im Vakuum. Das erhaltene erfindungsgemäße Brausegranulat wird gegebenenfalls mit weiteren Bestandteilen der Zusammensetzung in Stickpacks gefüllt. Die Dosierung der Brauseformulierung spielt für die Erfindung eine wichtige Rolle. Auf der einen Seite muss genug Brausemittel vorhanden sein, um eine spürbare Kohlendioxidfreisetzung zu ermöglichen, die das angenehm frische Mundgefühl und die Geschmacksmaskierung bewirkt. Auf der anderen Seite soll die Zusammensetzung im Mundraum nicht stark schäumen, weil dies von der Mehrzahl der Patienten ebenfalls nicht als angenehm empfunden wird. Der Anteil der Brausemittels an der Zusammensetzung macht daher idealerweise 3 - 60 % des Gesamtgewichts aus, bevorzugt 5 - 45 % und besonders bevorzugt 10-30%.

Verschiedene dem Fachmann bekannte Methoden existieren, die es erlauben phosphatbindende Wirkstoffe in ein Granulat einzuarbeiten. Bevorzugt liegt hierbei der Wirkstoff gegebenenfalls unter Zusatz von Hilfsstoffen selbst als Granulat vor, das die granulierte Endzusammensetzung darstellt oder Bestandteil von ihr ist. Bei Einarbeitung in ein Granulat können sowohl vorgranulierte Wirkstoffe als auch pulverförmige Wirkstoffe, die dann in Folgeschritten granuliert werden, eingesetzt werden. Die Arzneiformen können Hilfsstoffe enthalten, die sowohl in die Granulatstruktur eingebunden als auch den Granulatpartikeln pulverförmig zugemischt sein können. Als Hilfsstoffe kommen insbesondere Füllstoffe, Süßmittel, Säuerungsmittel, Aromen, Überzugsmittel, Fließregulierungsmittel und Trennmittel zum Einsatz. Als Granulationsverfahren können insbesondere und ohne andere Verfahren auszuschließen Feuchtgranulierung, Trockengranulierung und Schmelzgranulierung eingesetzt werden. Bei der Feuchtgranulierung sind wässrige Verfahren aus Umwelt- und Arbeitsschutzgründen bevorzugt, Verfahren mit organischen Lösungsmitteln aber auch möglich. Für Feucht- und Schmelzgranulierung sind Wirbelschicht- und Schnellmischerverfahren (z.B. Diosna Mischer oder Granumat der Fa. Bohle) bevorzugt, alternative Verfahren aber auch möglich. Es werden dabei Granulierungsverfahren bevorzugt, die zu einem möglichst dichten Granulat führen, um das Volumen der Einnahmedosis zu verkleinern. Bevorzugt werden weiterhin Granulate mit einer glatten, wenig porösen Oberfläche, die ein gleichmäßiges Anfeuchten durch Speichel ermöglichen und durch die Glätte ein angenehmes Mundgefühl vermitteln. Bevorzugte Granulierungsverfahren sind die Feuchtgranulierung der Wirkstoffe mit Bindemitteln mit anschließender Trocknung im Wirbelbett und die Schmelzgranulierung.

In bevorzugten Ausführungsformen werden dem Wirkstoff bei der Granulierung weitere vorteilhafte Hilfsstoffe beigefügt. Verschiedene Hilfsstoffe - wie Füller, Trennmittel, Bindemittel, Sprengmittel, Fließregulierungsmittel, Süßstoffe und/oder Aromen - werden bei der Granulierung aus technischen und pharmazeutischen Gründen verwendet und sind dem Fachmann wohl bekannt. Im Rahmen der Erfindung tragen die Hilfsstoffe aber idealerweise zusätzlich dazu bei, das Mundgefühl und den Geschmack des Granulats zu verbessern.

Als Füllstoffe kommen alle pharmazeutisch üblichen Tabletten- und Granulatfüllstoffe in Frage, insbesondere inerte Verbindungen wie Cellulosen, Stärken und/oder Lactose. Im Rahmen der Erfindung werden bevorzugt Zucker wie Saccharose, Glucose oder Fructose eingesetzt, die den Geschmack der Formulierung verbessern und somit gleichzeitig als Süßstoff fungieren. Besonders bevorzugt werden Zuckeralkohole eingesetzt, weil diese einerseits den unangenehmen Geschmack der Wirkstoffe durch angenehmen süßen Eigengeschmack maskieren und andererseits mit Speichel leicht anzufeuchten sind. Das führt zu dem gewünschten Effekt vermehrter Speichelbildung und trägt zu einem angenehmen kühlen Mundgefühl bei. Im Rahmen der Erfindung können bevorzugt Zuckeralkohole, ausgewählt aus der Gruppe Mannitol, Inositol, Erythritol, Lacitol, Xylitol, Maltitol, Malbitol, Sorbitol, Inulin und Isomalt, eingesetzt werden.

Die bereits zuvor genannten Zuckeralkohole können auch als bevorzugte Bindungsmittel in der Schmelzgranulierung eingesetzt werden. Andere im Rahmen der Erfindung bevorzugte Bindemittel bei der Granulierung mit gleichzeitigen geschmacksmaskierenden Eigenschaften sind: Cyclodextrine, Algininsäure, Eudragit E-100, Polacrillin, Microkristalline Cellulose, Bienenwachs, Glycerolester, Triglyceride, Polyglycerolester langkettiger Fettsäuren, PEG, Fettalkohol-PEG-Ester, Fettalkohol-PEG-Ether, PVP und Derivate, Polyacrylsäuren, Polyacrylate, Polymethacrylate, und beliebige Kombinationen davon. Besonders bevorzugt sind niedrig schmelzende Bindungsmittel, die eine Granulation bei niedrigen Temperaturen erlauben und Bindungsmittel, die gleichzeitig hydrophil sind oder gar zur Bildung von Hydrogelen neigen, weil so ein besseres Mundgefühl vermittelt wird. Zur zusätzlichen Süßung können auch Süßstoffe wie z.B. Aspartam, Saccharin, Cyclamat, Acesulfam, Neohesperidin, Trehalose, Alitam, Dihydroalcon, Taumatin und Sucralose eingesetzt werden, die neben der Süßung auch zur Speichelflussanregung führen können. Besonders der Zusatz von Saccharin ist bevorzugt, weil insbesondere durch Saccharin das fruchtige Aroma von Geschmacksstoffen unterstrichen wird und weil Saccharin von Probanden als besonders angenehm im Mundgefühl empfunden wurde.

Neben einer reinen Süßung werden auch Aromen und Geschmacksstoffe zur Geschmacksmaskierung, Erzeugung eines angenehmen Geschmacks und Vermehrung der Speichelbildung eingesetzt. Bevorzugt werden hierbei Fruchtaromen und besonders bevorzugt Zitrusfruchtaromen eingesetzt, weil diese einen besonders frischen Geschmack und ein kühles Mundgefühl vermitteln. Aus den gleichen Gründen sind auch Minzaromen bevorzugt.

Als Fließregulierungsmittel kommen solche Stoffe in Frage, die ein leichtes und gleichmäßiges Fließen der Granulate ermöglichen wie insbesondere hochdisperses Siliciumdioxid, Gefälltes Siliciumdioxid und Talkum.

Als Trennmittel eignen sich Fettsäuren und ihre Salze wie Stearinsäure, Magnesiumstearat, Calciumstearat, Behensäure und Calciumbehenat, Fettalkohole wie Stearylakohol, Fette wie Gehärtete Triglyceride und Gehärtetes Rizinusöl, Natriumfumarylstearat, Polyethylenglykol mit einem Molekulargewicht >1500 Dalton sowie Talkum. Die Wirkstoffgranulate werden geschmacksmaskierend überzogen. Dem Fachmann sind viele Beschichtungen und Beschichtungsverfahren für geschmacksmaskierende Überzüge bekannt. Für geschmacksmaskierende Beschichtungen im Sinne der Erfindung eigenen sich zum Beispiel folgende Materialien:
Celluloseacetat oder Celluloseacetatbutyrat mit PVP, Eudragit RD 100 mit Carboxymethylcellulose, Polyvinylalkohol und Polyethylenglykolcopolymer, Eudragit 100 und PVP, Polyvinylacetat und pharmazeutisch akzeptable hydrophile Polymere, Eudragit E100 und pharmazeutisch akzeptable Säuren, Polyacrylate, Polymethacrylate, Polymethacrylester, Kombinationen von magensaftlöslichen Polymeren mit hydrophilen gelbildenden Polymeren, wie Polyvinylacetat oder Polyvinylpyrrolidon (PVP).

Bevorzugtes Beschichtungsverfahren ist die Sprühbeschichtung im Wirbelbett. Prinzipiell sind hier sowohl Top-Spray als auch Bottom-Spray Verfahren üblich. Bevorzugt werden für das überziehen von Partikeln und Granulaten davon die Bottom-Spray-Verfahren. Typische Vertreter dieser Geräte werden z.B. von den Firmen Glatt, Aeromatic, Diosna und Bohle gebaut. Für das Bottom-Spray Verfahren wird ein sogenannter Wurster-Einsatz bevorzugt. Ein weiterer bevorzugter Typ eines Bottom-Spray Wirbelbettbeschichtungsgerätes ist der Kugelcoater bzw. der Mycrolab, Unilab, Pilotlab Familie bzw. HDGC der Firma Oyster Hüttlin. Wie dem Fachmann bekannt ist, erfolgt die Beschichtung in all diesen Systemen, indem eine Lösung bzw. Suspension eines Polymers in Wasser und/oder einem organischen Lösungsmittel mittels einer Düse auf ein durch eingeleitete, meistens erwärmte Luft erzeugtes Wirbelbett von Partikeln bzw. Granulaten oder Pellets aufgetragen und getrocknet wird.

Neben klassischen Verfahren, bei denen die Beschichtung in Wasser oder organischem Lösungsmittel gelöst ist und anschließend im Wirbelbett getrocknet wird, kommt das Verfahren der Schmelzbeschichtung in Frage und ist besonders bevorzugt. Bei dem Verfahren der Schmelzbeschichtung kommen niedrig schmelzende Bindungsmittel zum Einsatz, in deren Schmelze weitere Substanzen gelöst oder suspendiert sind. Die Schmelze wird im Wirbelbett aufgesprüht und die Beschichtung erhärtet sich bei Erkaltung. Idealerweise werden Bindemittel verwendet, die bereits für sich selbst geschmacksmaskierende Wirkung entfalten und im Mund als angenehm empfunden werden, wie zum Beispiel lipophile Substanzen, ausgewählt aus der Gruppe der Lipide, Wachse, Glycerolester, Triglyceride, Polyglycerolester langkettiger Fettsäuren, PEG, Fettalkohol-PEG-Ester, Fettalkohol-PEG-Ether und PVP und Derivate, Polyacrylsäuren, Polyacrylate, Polymethacrylate, und Kombinationen davon. Besonders bevorzugt sind niedrig schmelzende Bindemittel, die eine Granulierung bei niedrigen Temperaturen erlauben und Bindemittel, die gleichzeitig hydrophil sind oder gar zur Bildung von Hydrogelen neigen, weil so ein besseres Mundgefühl vermittelt wird.

Bevorzugt wird der Beschichtung noch ein Oberflächenmodifikationsmittel beigemischt, was eine glatte leicht zu befeuchtende Oberfläche erzeugt und somit das Mundgefühl zusätzlich verbessert. Beispiele für Oberflächenmodifikationsmittel sind Sorbitanfettsäureester, Polyoxyethylalkylester, Polyoxyethylalkylether Polyoxyethylsorbitanfettsäureester, Polyoxyethylstearate sowie Copolymere davon. Das Brausemittel sollte im Wesentlichen nicht im Wirkstoffgranulat enthalten sein, weil der geschmacksmaskierende Überzug die Befeuchtung des Brausemittels durch Speichel einerseits erschwert und damit die Freisetzung von Kohlendioxid beeinträchtigt und andererseits durch eventuell entstehendes Kohlendioxid der geschmacksmaskierende Überzug zumindest teilweise aufgebrochen werden kann. In einem solchen Fall kann das Brausemittel in Form eines weiteren Granulats oder als Pulver der Zusammensetzung beigemischt werden. In einer weiteren Ausführungsform kann das Brausemittel in der äußeren Schicht der Beschichtung enthalten sein; dabei kann es sich um die gleiche Schicht wie die geschmacksmaskierende Beschichtung handeln oder um eine weitere Schicht.

Das gleiche wie für das Brausemittel gilt auch für Süßstoffe und Aromen, die Ihre geschmacksverbessernden Eigenschaften im Kern des beschichteten Wirkstoffgranulats nicht entfalten können. Auch Süßstoffen und Aromen werden bevorzugt in Form eines weiteren Granulats oder als Pulver zugegeben, wobei es sich auch um das Granulat, das das Brausemittel enthält, handeln kann oder sie sind besonders bevorzugt Bestandteil einer äußeren Beschichtung.

Die Zusammensetzung wird in Form eines rieselfähigen Granulats zur Verfügung gestellt. Die Kautablette bietet die Vorteile einer leichten Applizierbarkeit und der genauen Dosierbarkeit als Tablette. Dennoch wird im Rahmen der Erfindung die Form des rieselfähigen Granulats verwendet. Ein Grund dafür ist, dass die Kautablette zerkaut werden muss. Damit können zum einen geschmacksmaskierende Überzüge zerstört werden und zum anderen bewirkt das Zerkauen eine großräumige Verteilung im Mundraum und ein mögliches Anheften an den Zähnen, was ein schnelles quantitatives Abschlucken erschwert. Das Granulat kann direkt auf die Zunge appliziert und dort gelöst werden, dabei kommt es zu einer vergleichsweise geringen Verteilung der Partikel im Mundraum zwischen Zunge und Gaumen und das Granulat kann schnell quantitativ abgeschluckt werden. Es hat sich im Rahmen der Erfindung herausgestellt, dass Partikel in einer durchschnittlichen Partikelgröße von 100 - 3500 µm, bevorzugt 250 - 2500 µm und besonders bevorzugt 400 - 2000 µm, für diese Anwendung als besonders angenehm empfunden werden. (Die durchschnittliche Partikelgröße kann nach der Rüttelsiebmethode DIN53477 ermittelt werden.) Partikel dieser Größe vereinen sich häufig im Mundraum zu einer partikulär breiartigen Konsistenz, die leicht abzuschlucken ist. Größere Partikel verhindern die Formung dieser vorteilhaften breiartigen Konsistenz, wogegen kleinere Partikel im Mundraum als staubig empfunden werden. Kleinere Partikel adsorbieren wegen Ihrer insgesamt größeren Oberfläche mehr Speichel, es wird mehr Speichel zur Anlösung benötigt, was den unerwünschten Effekt der Trockenheit im Mund fördert.

Der Phosphatbinder in einer täglichen Gesamtdosis von 40 - 10000 mg kann als Einzeldosis oder als Mehrfachdosis abgefüllt werden. Dabei sollte die Tagesdosis auf die Mahlzeiten, üblicherweise drei, aufgeteilt werden und mit der Mahlzeit eingenommen werden. Die genaue Dosierung wird bevorzugt im Verlauf der Behandlung den Phosphatwerten des Patienten angepasst. Es bietet sich somit an, die gebräuchlichste Dosis pro Mahlzeit einzeln abzupacken. Patienten, mit einem niedrigen Bedarf an Phosphatbinder würde dann weniger als den Gesamtinhalt einer Packung pro Mahlzeit einnehmen beziehungsweise den Inhalt einer Packung auf mehrere Mahlzeiten aufteilen. Patienten mit einem hohen Bedarf können mehr als den Inhalt einer Packung zu sich nehmen.

Für einen Phosphatbinder auf Calciumbasis wird bevorzugt 70 - 700 mg Calcium und 0 - 300 mg Magnesium, bezogen auf das Gewicht der Metallionen, bevorzugt 100 - 450 mg Calcium und 50 - 240 mg Magnesium und ganz besonders bevorzugt 150 - 300 mg Calcium und 80 - 160 mg Magnesium, einzeln abgepackt.

Für einen Phosphatbinder auf lonenaustauscherbasis wird bevorzugt 150 - 3000 mg des Wirkstoffs, bevorzugt 300 - 2500 mg und ganz besonders bevorzugt 600 - 1700 mg, einzeln abgepackt.

Für einen Phosphatbinder auf Lanthanbasis wird bevorzugt 40 - 1000 mg Lanthan, bezogen auf das Gewicht der Metallionen, bevorzugt 50 - 750 mg und ganz besonders bevorzugt 80 - 500 mg, einzeln abgepackt.

Als Verpackung eignen sich für die erfindungsgemäßen Zubereitungen in Form eines Granulats insbesondere solche Verpackungen, die die zu verabreichende Dosis des Phosphatbinders enthalten und aus denen die Zubereitung direkt in den Mund, bevorzugt auf die Zunge, appliziert werden kann. Bevorzugt ist eine Beutelverpackung, besonders bevorzugt eine sogenannte Stickpack-Verpackung, in der die Zubereitung in eine schlauchförmige Verpackung eingeschlossen ist. Die schlauchförmige Verpackung wird bevorzugt hergestellt, indem das Material, bevorzugt eine Folie, um eine runde Führungshülse gelegt und an den sich berührenden Seiten des Materials verbunden wird. Der so entstandene Schlauch wird an seinem unteren Ende verschlossen, mit der Zubereitung, die bevorzugt durch die runde Führungshülse dosiert wird, befüllt und dann an seinem oberen Ende verschlossen. Im Falle eines Folienschlauchs erfolgt vorzugsweise das Verschweißen des oberen Teils Verpackung zusammen mit dem Verschweißen des unteren Teils der nachfolgenden Verpackung und der Trennung der beiden Verpackungen. Der Stickpack hat nach dem Aufreißen oder Aufschneiden an einer kurzen Seite des Beutels eine kleine Öffnung von bevorzugt weniger als 1 cm Durchmesser, aus der die Zubereitung bevorzugt der Schwerkraft folgend aus dem Beutel in den Mund appliziert werden kann. Das Granulat rieselt aus der Verpackung in den Mund, wobei die Zufuhr des Granulats bei größeren Füllmengen des Beutels portionsweise erfolgen kann. Kleinere Füllmengen können als eine Schüttung auf einmal appliziert werden.

Die Füllmenge eines Stickpacks orientiert sich an der Menge, die zum einen eine ausreichende Menge Wirkstoff enthält und zum anderen vom Patienten bei der Aufnahme in den Mundraum noch als angenehm empfunden wird. Daher enthält ein solches Stickpack bevorzugt 0,5 - 8 g der Gesamtzusammensetzung, besonders bevorzugt enthält das Stickpack 0,75 - 5 g der Zusammensetzung und ganz besonders bevorzugt 1 - 3,5 g.

### Beispiele

### Beispiel 1 (als Referenz)

Eine Granulatzubereitung mit 110 mg Calcium und 60 mg Magnesium pro Dosis kann auf folgende Art und Weise hergestellt werden:

| | |
|---|---|
| Calciumacetat (gemäß Anforderungen des Europäischen Arzneibuchs)* | 435 g |
| Schweres Magnesiumcarbonat (gemäß Anforderungen des Europäischen Arzneibuchs) | 235 g |
| Mannitol | 338 g |
| Natriumhydrogencarbonat | 67 g |
| Mononatriumdihydrogencitrat | 67 g |
| Citronensäure | 13g |
| Aspartam | 5 g |
| Orangen Aroma | 30 g |
| Hochdisperses Siliciumdioxid | 10 g |

| | |
|---|---|
| *Für wasserfreies Calciumacetat, für wasserhaltiges Calciumacetat muss jeweils die Einwaage in Bezug auf den Wassergehalt angepasst und mehr Calciumacetat eingewogen werden. Dieser Schritt ist für alle Beispiele mit Calciumacetat zu berücksichtigen. | |

Calciumacetat, schweres Magnesiumcarbonat, Natriumhydrogenphosphat und 75 % des Mannitols werden mittels einer Walzenkompaktierung und anschließenden Siebung durch ein 1,5 mm Sieb in ein Granulat überführt. Mononatriumhydrogencitrat, Citronensäure und die verbliebenen 25% des Mannitols werden mittels einer Walzenkompaktierung und anschließenden Siebung durch ein 1,5 mm Sieb ebenfalls in ein Granulat überführt.

Beide Granulate, Aspartam, Orangen Aroma und Hochdisperses Siliciumdioxid werden in einem Containermischer gemischt und anschließend mit einem Füllgewicht von 1200 mg in Stickpacks verpackt.

### Beispiel 2 (als Referenz)

Eine Granulatzubereitung mit 110 mg Calcium und 60 mg Magnesium pro Dosis kann ebenfalls auf folgende Art und Weise hergestellt werden:

**Brausegranulat:**

| | |
|---|---|
| Citronensäure | 75 g |
| Mononatriumcitrat | 75 g |
| Natriumhydrogencarbonat | 150 g |
| Natriumcyclamat | 20 g |
| Saccharosepalmitat | 2 g |
| Hydroxypropylmethylcellulose | 5 g |

**Übrige Bestandteile:**

| | |
|---|---|
| Calciumacetat (gemäß Anforderungen des Europäischen Arzneibuchs), granuliert (Firma Paul Lohmann) | 435 g |
| Schweres Magnesiumcarbonat (gemäß Anforderungen des Europäischen Arzneibuchs), granuliert mit ca. 10 % Maisstärke (Magnesiumcarbonat DC90 S/C Firma Paul Lohmann) | 262 g |
| Aspartam | 10 g |
| Sorbitol | 200 g |
| Xylitol | 136 g |
| Orangen-Aroma | 30 g |

Für das Brausegranulat werden Citronensäure, Mononatriumcitrat, Natriumhydrogencarbonat, Natriumcyclamat, Saccharosepalmitat und Hydroxypropylcellulose in einem Diosna.Mischer mit 1 l Behälter gemischt und mittels einer Düse im Mischer unter Rühren langsam mit 5 g Ethanol besprüht. Anschließend wird noch 10 min weitergranuliert. Dann wird die Masse auf ein Hordenblech ausgebreitet und 60 min in einem Vakuumtrockenschrank bei 30°C und einem Enddruck <50 mbar getrocknet.

Das so entstandene Brausegranulat wird durch ein 1 mm Sieb gepresst und mit den übrigen Bestandteilen in einem 5 l Kubusmischer gemischt.

Die Mischung wird in Portionen zu 1400 mg in ein Aluminiumstickpack eingeschweißt.

### Beispiel 3 (als Referenz)

Eine Granulatzubereitung mit 167 mg Calcium pro Dosis kann auf folgende Art und Weise hergestellt werden:

| | |
|---|---|
| Calciumacetat (gemäß Anforderungen des Europäischen Arzneibuchs), granuliert (Firma Paul Lohmann) | 660 g |
| Aspartam | 10 g |
| Sorbitol | 200 g |
| Xylitol | 100 g |
| Orangen-Aroma | 30 g |
| Fertiges Brausegranulat aus Beispiel 2 | 200 g |

Alle Bestandteilen werden in einem 5 I Kubusmischer gemischt. Die Mischung wird in Portionen zu 1200 mg in ein Aluminiumstickpack eingeschweißt.

### Beispiel 4

Eine Granulatzubereitung mit 167 mg Calcium pro Dosis kann ebenfalls auf folgende Art und Weise hergestellt werden:

**Geschmacksmaskierend überzogenes Calciumacetat:**

| | |
|---|---|
| Calciumacetat (gemäß Anforderungen des Europäischen Arzneibuchs), granuliert (Firma Paul Lohmann) | 1980 g |
| Glycerolpalmitostearat (Precirol® ATO 5, Firma Gattefosse) | 220 g |

**Weitere Bestandteile:**

| | |
|---|---|
| Aspartam | 30 g |
| Sorbitol | 600 g |
| Xylitol | 320 g |
| Orangen-Aroma | 100 g |
| Fertiges Brausegranulat aus Beispiel 2 | 650 g |

Das Calciumacetat wird in einem Wirbelschichtgerät (Unilab 05 der Forma Hüttlin) mit einer Zulufttemperatur von 35°C bewegt und mit einer auf 80°C erhitzten Schmelze von Glycerolpalmitostearat, die mittels auf 60°C erhitzter Sprühluft durch eine Sprühdüse von 1 mm mit einem Luftdruck von 1 bar vernebelt wird, mit einer Sprührate von 6 g pro Minute überzogen. Abschließend wird das überzogene Granulat im Kugelcoater für 10 min auf 25°C abgekühlt.

Das überzogene Calciumacetat wird durch ein 2 mm Sieb gegeben, mit den übrigen Bestandteilen in einem 10 l Kubusmischer gemischt und anschließend in Portionen zu 1300 mg in Aluminiumstickpacks eingeschweißt.

### Beispiel 5

Eine Granulatzubereitung mit 220 mg Calcium und 120 mg Magnesium pro Dosis kann auf folgende Art und Weise hergestellt werden:

| | |
|---|---|
| Geschmacksmaskierend überzogenes Calciumacetat aus Beispiel 4 (Calciumacetatgehalt 90%) | 1450 g |
| Schweres Magnesiumcarbonat (gemäß Anforderungen des Europäischen Arzneibuchs), granuliert mit ca. 10 % Maisstärke (Magnesiumcarbonat DC90 S/C Firma Paul Lohmann) | 785 g |
| Aspartam | 30 g |
| Sorbitol | 600 g |
| Xylitol | 400 g |
| Orangen-Aroma | 100 g |
| Fertiges Brausegranulat aus Beispiel 2 | 625 g |

Alle Bestandteile werden in einem 10 I Kubusmischer gemischt und in Portionen zu 2660 mg in Aluminiumstickpacks eingeschweißt.

### Beispiel 6

Eine Granulatzubereitung mit 220 mg Calcium und 120 mg Magnesium pro Dosis kann auf folgende Art und Weise hergestellt werden:

| | |
|---|---|
| Geschmacksmaskierend überzogenes Calciumacetat aus Beispiel 4 (Calciumacetatgehalt 90%) | 1450 g |
| Schweres Magnesiumcarbonat (gemäß Anforderungen des Europäischen Arzneibuchs), granuliert mit ca. 10 % Maisstärke (Magnesiumcarbonat DC90 S/C Firma Paul Lohmann) | 785 g |
| Aspartam | 30 g |
| Sorbitol | 550 g |
| Xylitol | 400 g |
| Natriumcarboxymethylcellulose | 50 g |
| Orangen-Aroma | 100 g |
| Fertiges Brausegranulat aus Beispiel 2 | 625 g |

Alle Bestandteile werden in einem 10 I Kubusmischer gemischt und in Portionen zu 2660 mg in Aluminiumstickpacks eingeschweißt.

### Beispiel 7

Die fertige Mischung aus Beispiel 4 kann zur Herstellung unterschiedlicher Calciumdosierungen in folgenden Portionen in Stickpacks von entsprechendem Füllvolumen (anpassbar durch Breite und vor allem Länge der Stickpacks) abgefüllt werden:

| Calciumdosis | Entsprechende Masse Calciumacetat (wasserfrei) | Füllmenge der Stickpacks |
|---|---|---|
| 50 mg | 198 mg | 389 mg |
| 100 mg | 395 mg | 778 mg |
| 150 mg | 593 mg | 1168 mg |
| 200 mg | 791 mg | 1557 mg |
| 250 mg | 988 mg | 1946 mg |
| 300 mg | 1186 mg | 2335 mg |
| 350 mg | 1384 mg | 2725 mg |
| 400 mg | 1582 mg | 3114 mg |
| 500 mg | 1977 mg | 3892 mg |

### Beispiel 8

Eine Granulatzubereitung mit 220 mg Calcium und 110 mg Magnesium pro Dosis kann auf folgende Art und Weise hergestellt werden:

| | |
|---|---|
| Geschmacksmaskierend überzogenes Calciumacetat aus Beispiel 4 (Calciumacetatgehalt 90%) | 1450 g |
| Schweres Magnesiumcarbonat (gemäß Anforderungen des Europäischen Arzneibuchs), granuliert mit ca. 10 % Maisstärke (Magnesiumcarbonat DC90 S/C Firma Paul Lohmann) | 720 g |
| Aspartam | 30 g |
| Sorbitol | 550 g |
| Xylitol | 375 g |
| Natriumcarboxymethylcellulose | 50 g |
| Orangen-Aroma | 100 g |
| Fertiges Brausegranulat aus Beispiel 2 | 625 g |

Alle Bestandteile werden in einem 10 I Kubusmischer gemischt und in Portionen zu 2600 mg in Aluminiumstickpacks eingeschweißt.

### Beispiel 9

Die fertige Mischung aus Beispiel 8 kann zur Herstellung unterschiedlicher Calcium- und Magnesiumdosierungen, jeweils im Verhältnis 2:1 (Ca:Mg) in folgenden Portionen in Stickpacks von entsprechendem Füllvolumen (anpassbar durch Breite und vor allem Länge der Stickpacks) abgefüllt werden:

| Calciumdosis | Magnesiumdosis | Füllmenge der Stickpacks |
|---|---|---|
| 50 mg | 25 mg | 591 mg |
| 100 mg | 50 mg | 1182 mg |
| 150 mg | 75 mg | 1773 mg |
| 200 mg | 100 mg | 2364 mg |
| 250 mg | 125 mg | 2955 mg |
| 300 mg | 150 mg | 3545 mg |
| 350 mg | 175 mg | 4136 mg |

### Beispiel 10

Die fertige Mischung aus Beispiel 4 kann durch Zumischen von Magnesiumcarbonat zur Herstellung unterschiedlicher Calciumdosierungen mit unterschiedlichen Dosierungsverhältnissen von Calcium zu Magnesium genutzt werden. Dadurch ergeben sich folgende Füllmengen in Stickpacks von entsprechendem Füllvolumen (anpassbar durch Breite und vor allem Länge der Stickpacks) abgefüllt werden:

| Ca:Mg Verhältnis | Calciumdosis | Magnesiumdosis | Entsprechend einzusetzende Masse der Mischung aus Beispiel 4 pro Stickpack für Calcium | Entsprechend einzusetzende Masse Magnesiumcarbonat DC90 S/C für Magnesium pro Stickpack | Füllmenge der Stickpacks |
|---|---|---|---|---|---|
| 4:1 | 100 mg | 25 mg | 778 mg | 28 mg | 806 mg |
| 2:1 | 100 mg | 50 mg | 778 mg | 56 mg | 834 mg |
| 1:1 | 100 mg | 100 mg | 778 mg | 111 mg | 889 mg |
| 3:1 | 150 mg | 50 mg | 1168 mg | 56 mg | 1224 mg |
| 2:1 | 150 mg | 75 mg | 1168 mg | 83 mg | 1251 mg |
| 1,5:1 | 150 mg | 100 mg | 1168 mg | 111 mg | 1279 mg |
| 4:1 | 200 mg | 50 mg | 1557 mg | 56 mg | 1613 mg |
| 2:1 | 200 mg | 100 mg | 1557 mg | 111 mg | 1668 mg |
| 1,33:1 | 200 mg | 150 mg | 1557 mg | 167 mg | 1724 mg |
| 1:1 | 200 mg | 200 mg | 1557 mg | 222 mg | 1779 mg |
| 5:1 | 250 mg | 50 mg | 1946 mg | 56 mg | 2002 mg |
| 2,5:1 | 250 mg | 100 mg | 1946 mg | 111 mg | 2057 mg |
| 2:1 | 250 mg | 125 mg | 1946 mg | 139 mg | 2085 mg |
| 1,67:1 | 250 mg | 150 mg | 1946 mg | 167 mg | 2113 mg |
| 1:1 | 250 mg | 250 mg | 1946 mg | 278 mg | 2224 mg |
| 3:1 | 300 mg | 100 mg | 2335 mg | 111 mg | 2446 mg |
| 2:1 | 300 mg | 150 mg | 2335 mg | 167 mg | 2502 mg |
| 1,5:1 | 300 mg | 200 mg | 2335 mg | 222 mg | 2557 mg |
| 1:1 | 300 mg | 300 mg | 2335 mg | 333 mg | 2668 mg |

### Beispiel 11

Eine Granulatzubereitung mit 167 mg Calcium pro Dosis kann ebenfalls auf folgende Art und Weise hergestellt werden:

**Geschmacksmaskierend überzogenes Calciumacetat:**

| | |
|---|---|
| Calciumacetat (gemäß Anforderungen des Europäischen Arzneibuchs), granuliert (Firma Paul Lohmann) | 1980 g |
| Basisches Butyliertes Methacrylat Copolymer, Ph. Eur. (Eudragit EPO®, Firma Evonik) | 400 g |
| Natriumdodecylsulfat | 40 g |
| Stearinsäure | 60 g |
| Talkum | 160 g |

**Weitere Bestandteile:**

| | |
|---|---|
| Aspartam | 30 g |
| Sorbitol | 600 g |
| Xylitol | 320 g |
| Orangen-Aroma | 100 g |
| Fertiges Brausegranulat aus Beispiel 2 | 810 g |

Zuerst wird eine Filmüberzugssuspension mit einem Überschuss von 25% auf die oben angegeben Bestandteile des Überzugsmaterials. Der Überschuss deckt die Sprühverluste während der Herstellung ab, so dass die geplante Menge eines Überzugs von 20% Methacrylatpolymer aufgetragen werden kann. Dazu wird zunächst 50 g Natriumdodecylsulfat in 3,5 I gereinigtem Wasser von Raumtemperatur (ca. 20°C) aufgelöst. Nach 5 min werden 75 g Stearinsäure in der Lösung unter weiterem Rühren mit einem UltraTurrax dispergiert. Nach nochmals 5 min werden 500 g Eudragit EPO unter weiterem Rühren in der Dispersion verteilt und 30 min intensiv mit dem UltraTurrax weiter dispergiert. Parallel werden 200 g Talkum unter Rühren mit einem UltraTurrax in 1,5 I gereinigtem Wasser von Raumtemperatur (ca. 20°C) dispergiert und weitere 20 min intensiv gerührt. Beide Dispersionen werden gemischt und dann mittels eines Wirbelschichtgeräts mit Wurster-Einsatz (Glatt GCPG2 LabSystem) im Bottom-Spray-Verfahren auf das granulierte Calciumacetat aufgetragen. Der Auftrag erfolgt in zwei Teilchargen, für die jeweils die Hälfte der Materialien eingesetzt wird. Die Zulufttemperatur wird auf ca. 50°C eingestellt, so dass sich während des Befilmens eine Produkttemperatur von ca. 30°C einstellt. Die Suspension wird mittels einer 1,2 mm Düse mit einem Luftdruck von ca. 1,5 bar und einer Zuführgeschwindigkeit von 10 g pro min auf das Calciumacetat aufgetragen. Der Prozess wird beendet, wenn das Gewicht des Granulats um ca. 33% zugenommen hat. Das überzogene Granulat aus beiden Teilchargen wird über ein 2 mm Sieb gegeben und mit den übrigen Bestandteilen in einem 10 l Kubusmischer gemischt und anschließend in Portionen zu 1500 mg in Aluminiumstickpacks eingeschweißt.

### Beispiel 12

Eine Granulatzubereitung mit 110 mg Calcium und 60 mg Magnesium pro Dosis kann auf folgende Art und Weise hergestellt werden:

| | |
|---|---|
| Geschmacksmaskierend überzogenes Calciumacetat aus Beispiel 11 (Calciumacetatgehalt 75%) | 1740 g |
| Schweres Magnesiumcarbonat (gemäß Anforderungen des Europäischen Arzneibuchs), granuliert mit ca. 10 % Maisstärke (Magnesiumcarbonat DC90 S/C Firma Paul Lohmann) | 785 g |
| Aspartam | 30 g |
| Sorbitol | 600 g |
| Xylitol | 400 g |
| Orangen-Aroma | 100 g |
| Fertiges Brausegranulat aus Beispiel 2 | 695 g |

Alle Bestandteile werden in einem 10 l Kubusmischer gemischt und in Portionen zu 1450 mg in Aluminiumstickpacks eingeschweißt.

### Beispiel 13

Eine Granulatzubereitung mit 220 mg Calcium und 120 mg Magnesium pro Dosis kann durch Abfüllen der fertigen Mischung aus Beispiel 12 in Portionen zu 2900 mg in Aluminiumstickpacks erfolgen.

### Beispiel 14 (als Referenz)

Eine Zubereitung mit 125 mg Lanthanum (=238,5 mg Lanthanhydroxycarbonat) pro Dosis kann auf folgende Art und Weise hergestellt werden:

| | |
|---|---|
| Lanthanhydroxycarbonat (Lanthangehalt 52,4%) | 715 g |
| Aspartam | 10 g |
| Sorbitol | 200 g |
| Xylitol | 100 g |
| Orangen-Aroma | 30 g |
| Fertiges Brausegranulat aus Beispiel 2 | 220 g |

Alle Bestandteilen werden in einem 5 I Kubusmischer gemischt. Die Mischung wird in Portionen zu 425 mg in ein Aluminiumstickpack eingeschweißt.

### Beispiel 15 (als Referenz)

Zubereitungen mit 250 mg, 500 mg, 750 mg und 1000 mg Lanthanum pro Dosis können durch Abfüllen der Mischung aus Beispiel 14 gemäß folgender Tabelle in Aluminiumstickpacks:

| Lanthanumdosis | Entsprechende Masse Lanthanhydroxycarbonat | Füllmenge der Stickpacks mit fertiger Mischung aus Beispiel 14 |
|---|---|---|
| 250 mg | 477 mg | 850 mg |
| 500 mg | 954 mg | 1700 mg |
| 750 mg | 1431 mg | 2550 mg |
| 1000 mg | 1908 mg | 3400 mg |

### Beispiel 16 (als Referenz)

Eine Zubereitung mit 800 mg Sevelamerhydrochlorid pro Dosis kann auf folgende Art und Weise hergestellt werden:

| | |
|---|---|
| Sevelamerhydrochlorid, getrocknet | 800 g |
| Aspartam | 10 g |
| Sorbitol | 200 g |
| Xylitol | 100 g |
| Orangen-Aroma | 40 g |
| Fertiges Brausegranulat aus Beispiel 2 | 250 g |

Alle Bestandteilen werden in einem 5 I Kubusmischer gemischt. Die Mischung wird in Portionen zu 1400 mg in ein Aluminiumstickpack eingeschweißt.

### Beispiel 17 (als Referenz)

Zubereitungen mit 1600 mg und 2400 mg Sevelamer pro Dosis können durch Abfüllen der Mischung aus Beispiel 16 gemäß folgender Tabelle in Aluminiumstickpacks:

| Sevelamerdosis | Füllmenge der Stickpacks mit fertiger Mischung aus Beispiel 16 |
|---|---|
| 1600 mg | 2800 mg |
| 2400 mg | 4200 mg |

### Beispiel 18 (als Referenz)

Eine Zubereitung mit 800 mg Sevelamerhydrochlorid pro Dosis kann auf folgende Art und Weise hergestellt werden:

| | |
|---|---|
| Sevelamerhydrochlorid, getrocknet | 800 g |
| Povidon K30 | 50 g |
| Aspartam | 10 g |
| Sorbitol | 200 g |
| Xylitol | 100 g |
| Orangen-Aroma | 40 g |
| Fertiges Brausegranulat aus Beispiel 2 | 250 g |

Povidon K30 wird in 150 ml eines Gemisches aus 90% Ethanol und 10% gereinigtem Wasser aufgelöst. Sevelamerhydrochlorid wird mit der Povidonlösung in einem Wirbelschichtgranulator (Glatt GCPG2 LabSystem) im Top-Spray-Verfahren granuliert. Das Granulat wird durch ein 1 mm Sieb getrieben und mit den übrigen Bestandteilen werden in einem 5 I Kubusmischer gemischt. Die Mischung wird in Portionen zu 1450 mg in ein Aluminiumstickpack eingeschweißt.

## Patentansprüche

1. Eine pharmazeutische Zusammensetzung in Form eines rieselfähigen Granulats, enthaltend mindestens eine phosphatbindende Substanz und mindestens ein Brausemittel, das ein Carbonat und eine feste, organische, essbare Säure oder deren saures Salz enthält, zur Verwendung in der Behandlung von Hyperphosphatämie bei niereninsuffizienten Patienten, wobei das Granulat oral ohne den Zusatz von Wasser appliziert wird, dadurch charakterisiert, dass die phosphatbindende Substanz ein Calciumacetat, ein Ionenaustauscherpolymer, eine Lanthanverbindung oder eine Eisenverbindung ist und mit einer geschmacksmaskierenden Beschichtung versehen ist.

2. Zusammensetzung nach Anspruch 1 zur Verwendung in der Behandlung von Hyperphosphatämie bei niereninsuffizienten Patienten, dadurch charakterisiert, dass das Carbonat ein Alkali- oder Erdalkalicarbonat oder ein Alkali- oder Erdalkalihydrogencarbonat ist.

3. Zusammensetzung nach Anspruch 3 zur Verwendung in der Behandlung von Hyperphosphatämie bei niereninsuffizienten Patienten, dadurch charakterisiert, dass die Beschichtung mit einem Sprühbeschichtungsverfahren im Wirbelbett hergestellt wird.

4. Zusammensetzung nach Anspruch 3 zur Verwendung in der Behandlung von Hyperphosphatämie bei niereninsuffizienten Patienten, dadurch charakterisiert, dass sich bei der Beschichtung um eine Schmelzbeschichtung handelt.

5. Zusammensetzung nach Anspruch 3 zur Verwendung in der Behandlung von Hyperphosphatämie bei niereninsuffizienten Patienten, dadurch charakterisiert, dass sich bei der Beschichtung um eine Sprühbeschichtung von Suspensionen und/oder Lösungen handelt.

6. Zusammensetzung nach einem der vorhergehenden Ansprüche zur Verwendung in der Behandlung von Hyperphosphatämie bei niereninsuffizienten Patienten, dadurch charakterisiert, dass die Zusammensetzung Magnesiumcarbonat enthält.

7. Zusammensetzung nach einem der vorhergehenden Ansprüche zur Verwendung in der Behandlung von Hyperphosphatämie bei niereninsuffizienten Patienten, dadurch charakterisiert, dass das Brausemittel in einer Beschichtung der phosphatbindenden Substanz enthalten ist.

8. Zusammensetzung nach einem der vorhergehenden Ansprüche zur Verwendung in der Behandlung von Hyperphosphatämie bei niereninsuffizienten Patienten, dadurch charakterisiert, dass sie mindestens einen Zuckeralkohol enthält.

9. Zusammensetzung nach einem der vorhergehenden Ansprüche zur Verwendung in der Behandlung von Hyperphosphatämie bei niereninsuffizienten Patienten dadurch charakterisiert, dass die phosphatbindende Substanz 15 - 80 % des Gewichts der Zusammensetzung ausmacht.

10. Zusammensetzung nach einem der vorhergehenden Ansprüche zur Verwendung in der Behandlung von Hyperphosphatämie bei niereninsuffizienten Patienten, dadurch charakterisiert, dass das Brausemittel 3 - 60 % des Gewichts der Zusammensetzung ausmacht.

11. Zusammensetzung nach einem der vorhergehenden Ansprüche zur Verwendung in der Behandlung von Hyperphosphatämie bei niereninsuffizienten Patienten, dadurch charakterisiert, dass der Mundraum nach der oralen Applikation der Zusammensetzung mit Wasser nachgespült wird.

12. Zusammensetzung nach einem der vorhergehenden Ansprüche zur Verwendung in der Behandlung von Hyperphosphatämie bei niereninsuffizienten Patienten, dadurch charakterisiert, dass sie in einer Dosierung von drei oder vier mal täglich 0,5 - 5 g, bevorzugt mit den Mahlzeiten, eingenommen wird.

13. Zusammensetzung nach einem der vorhergehenden Ansprüche zur Verwendung in der Behandlung von Hyperphosphatämie bei niereninsuffizienten Patienten, dadurch charakterisiert, dass die durchschnittliche Partikelgröße des Granulats 100 - 3500 µm beträgt.

## Claims

1. A pharmaceutical composition in the form of free-flowing granules containing at least one phosphate-binding substance and at least one effervescent agent, also containing a carbonate and a solid edible organic acid or the acid salt thereof, for use in the treatment of hyperphosphatemia in renally insufficient patients, wherein the granules are administered orally without adding water,
**characterized in that**
the phosphate-binding substance is a calcium acetate, an ion exchange polymer, a lanthanum compound or an iron compound and provided with a coating to mask the taste.

2. The composition according to claim 1, for use in the treatment of hyperphosphatemia in renally insufficient patients,
**characterized in that**
the carbonate is an alkali carbonate or alkaline earth carbonate or an alkali or alkaline earth bicarbonate.

3. The composition according to claim 3, for use in the treatment of hyperphosphatemia in renally insufficient patients,
**characterized in that**
the coating is prepared by a spray coating method in a fluidized bed.

4. The composition according to claim 3, for use in the treatment of hyperphosphatemia in renally insufficient patients,
**characterized in that**
the coating is a melt coating.

5. The composition according to claim 3, for use in the treatment of hyperphosphatemia in renally insufficient patients,
**characterized in that**
the coating is a spray coating of suspensions and/or solutions.

6. The composition according to any one of the preceding claims, for use in the treatment of hyperphosphatemia in renally insufficient patients,
**characterized in that**
the composition contains magnesium carbonate.

7. The composition according to any one of the preceding claims, for use in the treatment of hyperphosphatemia in renally insufficient patients,
**characterized in that**
the effervescent agent is contained in a coating on the phosphate-binding substance.

8. The composition according to any one of the preceding claims, for use in the treatment of hyperphosphatemia in renally insufficient patients,
**characterized in that**
it contains at least one sugar alcohol.

9. The composition according to any one of the preceding claims, for use in the treatment of hyperphosphatemia in renally insufficient patients,
**characterized in that**
the phosphate-binding substance constitutes 15-80% by weight of the composition.

10. The composition according to any one of the preceding claims, for use in the treatment of hyperphosphatemia in renally insufficient patients,
**characterized in that**
the effervescent agent constitutes 3-60% by weight of the composition.

11. The composition according to any one of the preceding claims, for use in the treatment of hyperphosphatemia in renally insufficient patients,
**characterized in that**
the oral cavity is rinsed with water after oral administration of the composition.

12. The composition according to any one of the preceding claims, for use in the treatment of hyperphosphatemia in renally insufficient patients,
**characterized in that**
it is taken in a dose of three or four times daily 0.5-5 g, preferably with meals.

13. The composition according to any one of the preceding claims, for use in the treatment of hyperphosphatemia in renally insufficient patients,
**characterized in that**
the average particle size of the granules is 100-3500 µm.

## Revendications

1. Composition pharmaceutique sous forme d'un granulé fluide, contenant au moins une substance liant le phosphate et au moins un agent effervescent qui contient un carbonate et un acide stable organique comestible ou son sel acide, destiné à une utilisation dans le traitement de l'hyperphosphatémie chez des patients insuffisants rénaux, dans lequel le granulat est appliqué oralement sans ajout d'eau, **caractérisé en ce que** la substance liant le phosphate est un acétate de calcium, un polymère échangeur ionique, un composé de lanthane ou un composé de fer et est pourvu d'un revêtement masquant le goût.

2. Composition selon la revendication 1 destinée à être utilisée dans le traitement de l'hyperphosphatémie chez des patients insuffisants rénaux, **caractérisée en ce que** le carbonate est un carbonate alcalin ou alcalin terreux ou un carbonate d'hydrogène alcalin ou alcalin terreux.

3. Composition selon la revendication 3 destinée à être utilisée dans le traitement de l'hyperphosphatémie chez des patients insuffisants rénaux, **caractérisée en ce que** le revêtement est préparé par procédé de revêtement par pulvérisation dans un lit fluidisé.

4. Composition selon la revendication 3 destinée à être utilisée dans le traitement de l'hyperphosphatémie chez des patients insuffisants rénaux, **caractérisée en ce que** le revêtement est un revêtement à fusion.

5. Composition selon la revendication 3 destinée à être utilisée dans le traitement de l'hyperphosphatémie chez des patients insuffisants rénaux, **caractérisée en ce que** le revêtement est un revêtement par pulvérisation de suspensions et/ou de solutions.

6. Composition selon une des revendications précédentes destinée à être utilisée dans le traitement de l'hyperphosphatémie chez des patients insuffisants rénaux, **caractérisée en ce que** le revêtement contient du carbonate de magnésium.

7. Composition selon une des revendications précédentes destinée à être utilisée dans le traitement de l'hyperphosphatémie chez des patients insuffisants rénaux, **caractérisée en ce que** l'agent effervescent est contenu dans un revêtement de la substance liant le phosphate.

8. Composition selon une des revendications précédentes destinée à être utilisée dans le traitement de l'hyperphosphatémie chez des patients insuffisants rénaux, **caractérisée en ce qu'**elle contient au moins un alcool de sucre.

9. Composition selon une des revendications précédentes destinée à être utilisée dans le traitement de l'hyperphosphatémie chez des patients insuffisants rénaux, **caractérisée en ce que** la substance liant le phosphate représente 15 à 80 % du poids de la composition.

10. Composition selon une des revendications précédentes destinée à être utilisée dans le traitement de l'hyperphosphatémie chez des patients insuffisants rénaux, **caractérisée en ce que** l'agent effervescent représente 3 à 60 % du poids de la composition.

11. Composition selon une des revendications précédentes destinée à être utilisée dans le traitement de l'hyperphosphatémie chez des patients insuffisants rénaux, **caractérisée en ce que** l'espace buccal est rincé avec de l'eau après l'application orale de la composition.

12. Composition selon une des revendications précédentes destinée à être utilisée dans le traitement de l'hyperphosphatémie chez des patients insuffisants rénaux, **caractérisée en ce qu'**elle est absorbée dans un dosage de 0,5 à 5 g quatre fois par jour, de préférence avec les repas.

13. Composition selon une des revendications précédentes destinée à être utilisée dans le traitement de l'hyperphosphatémie chez des patients insuffisants rénaux, **caractérisée en ce que** la taille moyenne des particules de granulé est de 100 à 3500 µm.
